(19) Europäisches Patentamt
European Patent Office
Office européen des brevets

(11) EP 1 681 285 A1

(12) **EUROPEAN PATENT APPLICATION**
published in accordance with Art. 158(3) EPC

(43) Date of publication:
**19.07.2006 Bulletin 2006/29**

(21) Application number: **04792637.3**

(22) Date of filing: **20.10.2004**

(51) Int Cl.:
*C07C 271/20* (1990.01) *C07C 271/34* (1990.01)
*C07C 269/06* (1990.01) *C07D 277/68* (1985.01)
*C07D 307/85* (1985.01) *C07D 333/60* (1985.01)
*A01N 47/12* (1985.01)

(86) International application number:
**PCT/JP2004/015471**

(87) International publication number:
**WO 2005/042474 (12.05.2005 Gazette 2005/19)**

(84) Designated Contracting States:
**AT BE BG CH CY CZ DE DK EE ES FI FR GB GR HU IE IT LI LU MC NL PL PT RO SE SI SK TR**

(30) Priority: **31.10.2003 JP 2003372467**

(71) Applicant: **Mitsui Chemicals, Inc.**
**Tokyo 105-7117 (JP)**

(72) Inventors:
- **EBIHARA, Koichi,**
  **c/o Mitsui Chemicals, Inc.**
  **Chiba 2970017 (JP)**
- **MORIZANE, Kunihiko,**
  **c/o Mitsui Chemicals, Inc.**
  **Chiba 2970017 (JP)**
- **TOMURA, Naofumi,**
  **c/o Mitsui Chemicals, Inc.**
  **Chiba 2970017 (JP)**
- **EZAKI, Ryutaro,**
  **c/o Mitsui Chemicals, Inc.**
  **Chiba 2970017 (JP)**
- **YOSHIDA, Masako,**
  **c/o Mitsui Chemicals, Inc.**
  **Chiba 2970017 (JP)**
- **OSADA, Yuko**
  **Komae-shi, Tokyo 2010001 (JP)**

(74) Representative: **Stuart, Ian Alexander et al**
**MEWBURN ELLIS**
**York House**
**23 Kingsway**
**London WC2B 6HP (GB)**

(54) **DIAMINE DERIVATIVE, PROCESS FOR PRODUCING THE SAME, AND PLANT DISEASE CONTROL AGENT CONTAINING THE SAME AS ACTIVE INGREDIENT**

(57) It is an object of the present invention to provide a diamine derivative, a process for producing the same, and a plant disease control agent containing the above-described compound as an active ingredient.

According to the present invention, a diamine derivative represented by Formula (1), a process for producing the same, and a plant disease control agent containing the above-described compound as an active ingredient are provided.

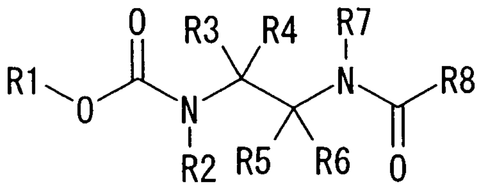 (1)

[In Formula, R1 represents, for example, a halogenated hydrocarbon having the carbon number of 1 to 6; R2 and R7 independently represent, for example, a hydrogen atom or a hydrocarbon having the carbon number of 1 to 6; R3 and R4 independently represent, for example, a hydrogen atom or a hydrocarbon which has the carbon number of 1 to 6 and which may be substituted, or represent a cycloalkyl group having the carbon number of 3 to 6 including a carbon atom bonding to R3 and R4; R5 and R6 independently represent, for example, a hydrogen atom or a hydrocarbon group having the carbon number of 1 to 6; and R8 represents an arylalkyl group which may be substituted, an aryl group which may be substituted, or a heteroaryl group which may be substituted.]

EP 1 681 285 A1

## Description

TECHNICAL FIELD

The present invention relates to new diamine derivatives, process for producing them, and plant disease control agents containing them as active ingredients.

BACKGROUND ART

The pest control plays an important role in cultivating paddy rice. In particular, a rice blast is a significant disease. Therefore, various plant disease control agents have been developed and put into use. However, every agent does not have a satisfactory plant disease control activity, nor is at a satisfactory level with respect to harm to useful crops. In recent years, fungi having resistance to chemical agents have appeared due to a heavy use of plant disease control agents and, sometimes, known chemical agents do not have satisfactory activities. Accordingly, a new plant disease control agent capable of controlling harmful fungi at a low agent concentration has been required. Japanese Unexamined Patent Application Publication No. 2003-096046 discloses diamine derivatives different from the compounds of the present invention and plant disease control agents containing them as active ingredients. However, in this document, there is no description on the usefulness of diamine derivatives including an oxycarbonyl group having a halogenated hydrocarbon.

Patent Document 1: Japanese Unexamined Patent Application Publication No. 2003-096046 (WO2003008372)

DISCLOSURE OF THE INVENTION

It is an object of the present invention to provide a diamine derivative exerting an excellent effect of controlling a rice blast.

Recently, the inventors of the present invention found out that diamine derivatives, in particular, diamine derivatives including an oxycarbonyl group having a halogenated hydrocarbon, exerted a high effect of controlling the rice blast. It was found out that this control effect was significantly higher than the effect exerted by other diamine derivatives disclosed in Japanese Unexamined Patent Application Publication No. 2003-096046, for example, and the present invention has been completed.

The present invention is as described below.

[1]. A diamine derivative represented by Formula (1):

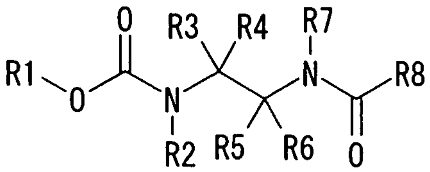

(1)

[In Formula, R1 represents a halogenated hydrocarbon having the carbon number of 1 to 6; R2 and R7 independently represent a hydrogen atom, a hydrocarbon having the carbon number of 1 to 6, or an acyl group; R3 and R4 independently represent a hydrogen atom, a hydrocarbon which has the carbon number of 1 to 6 and which may be substituted, or a heteroaryl group which may be substituted, or represent a cycloalkyl group having the carbon number of 3 to 6 including a carbon atom bonding to R3 and R4; R5 and R6 independently represent a hydrogen atom or a hydrocarbon having the carbon number of 1 to 6; and R8 represents an arylalkyl group which may be substituted, an aryl group which may be substituted, or a heteroaryl group which may be substituted.].

[2]. A plant disease control agent characterized in containing the diamine derivative according to the above-described [1] as an active ingredient.

[3]. A process for producing the diamine derivative according to the above-described [1], comprising reacting a compound represented by Formula (2):

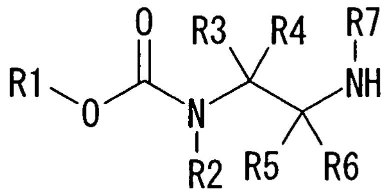

(2)

[In Formula, R1, R2, R3, R4, R5, R6, and R7 represent the same substances as those in [1]] with a compound represented by Formula (3):

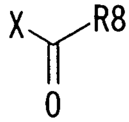

(3)

[In Formula, R8 represents the same substance as that in [1], and X represents a leaving group.].

[4]. A process for producing the diamine derivative according to [1], comprising condensing a compound represented by Formula (2) and a compound represented by Formula (4):

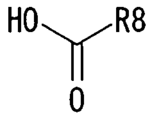

(4)

[In Formula, R8 represents the same substance as that in [1].].

[5]. A process for producing the diamine derivative according to the above-described [1], comprising reacting a compound represented by Formula (5):

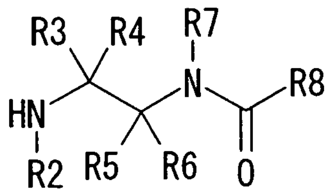

(5)

[In Formula, R2, R3, R4, R5, R6, R7, and R8 represent the same substances as those in [1].] with a compound represented by Formula (6):

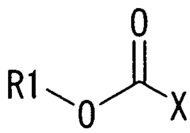

(6)

[In Formula, R1 represents the same substance as that in [1], and X represents a leaving group.].

The diamine derivative according to the present invention includes an oxycarbonyl group having a halogenated hydrocarbon and, thereby, exerts an excellent effect of controlling the rice blast.

BEST MODE FOR CARRYING OUT THE INVENTION

The present invention will be described below in detail.

In the diamine derivative represented by Formula (1) and the process for producing the same, typical examples of substituents include the following groups, although not limited to them. Examples of halogenated hydrocarbons having the carbon number of 1 to 6 include chlorinated alkyl groups, e.g., a chloromethyl group, a 2-chloroethyl group, a 2,2,2-trichloroethyl group, a 3-chloro-1-propyl group, and a 4-chloro-1-butyl group; fluorinated alkyl groups, e.g., a 2-fluoroethyl group, a 2,2,2-trifluoroethyl group, a 1,1,1,3,3,3-hexafluoro-2-propyl group, a 1,3-difluoro-2-propyl group, a 5-fluoro-1-pentyl group, a 6,6,6,5,5,4,4,3,3-nonafluoro-1-hexyl group, and a 1-ethoxy-2,2,2-trifluoroethyl group; brominated alkyl groups, e.g., a 2-bromoethyl group and a 1,3-dibromo-2-propyl group; iodized alkyl groups, e.g., a 2-iodoethyl group; alkyl groups containing at least two types of halogen, e.g., a 3-bromo-1,1,1-trifluoro-2-propyl group; chlorinated cycloalkyl groups, e.g., a chlorocyclopropyl group, a 2-chloro cyclobutyl group, a 2-chlorocyclopentyl group, a 2-chlorocyclohexyl group, a 3-chlorocyclohexyl group, and a 4-chlorocyclohexyl group; fluorinated cycloalkyl groups, e.g., a 2-fluorocyclohexyl group and a 2,2,3,3-tetrafluorocyclopropyl group; brominated cycloalkyl groups, e.g., a 2-bromocyclohexyl group; iodized cycloalkyl groups, e.g., a 2-iodocyclohexyl group; chlorinated alkenyl groups, e.g., a 2-chloro-2-propenyl group and a 5-chloro-4-pentenyl group; fluorinated alkenyl groups, e.g., a 4,4,4-trifluoro-2-butenyl group and a 6,6,6-trifluoro-5-hexenyl group; chlorinated cycloalkenyl groups, e.g., a 2-chloro-2-cyclopropenyl group, a 3-chloro-3-cyclopentenyl group, and a 2-chloro-2-cyclohexenyl group; and fluorinated cycloalkenyl groups, e.g., a 2-fluoro-2-cyclobutenyl group.

Examples of hydrocarbons having the carbon number of 1 to 6 include a methyl group, an ethyl group, a propyl group, a butyl group, a pentyl group, a hexyl group, a cyclopropyl group, a cyclobutyl group, a cyclopentyl group, a cyclohexyl group, a vinyl group, a propenyl group, a butenyl group, a pentenyl group, a hexenyl group, a cyclopropenyl group, a cyclobutenyl group, a cyclopentenyl group, a cyclohexenyl group, an ethynyl group, a propynyl group, a butynyl group, a pentynyl group, a hexynyl group, a phenyl group, and a naphthyl group. Examples of heteroaryl groups include a pyridyl group, a pyrimidyl group, a thienyl group, a furanyl group, a pyrazolyl group, an imidazolyl group, an isothiazolyl group, an isoxazolyl group, an indolyl group, a quinolyl group, a benzofuranyl group, a benzothienyl group, a benzoxazolyl group, a benzisoxazolyl group, a benzimidazolyl group, a benzothiazolyl group, and a benzisothiazolyl group. Examples of acyl groups include alkylcarbonyl groups, e.g., an acetyl group; and arylcarbonyl group, e.g., a benzoyl group. Examples of substituents in the aryl group and the heteroaryl group include alkyl groups, e.g., a methyl group, an ethyl group, a propyl group, and a butyl group; cycloalkyl groups, e.g., a cyclopropyl group, a cyclobutyl group, a cyclopentyl group, and a cyclohexyl group; halogenated alkyl groups, e.g., a trifluoromethyl group, a difluoromethyl group, a bromodifluoromethyl group, and a trifluoroethyl group; alkoxy groups, e.g., a methoxy group, an ethoxy group, a propoxy group, and a butoxy group; halogenated alkoxy groups, e.g., a trifluoromethoxy group, a difluoromethoxy group, and a trifluoroethoxy group; alkoxycarbonyl groups, e.g., a methoxycarbonyl group, an ethoxycarbonyl group, a propoxycarbonyl group, and a butoxycarbonyl group; aryloxycarbonyl groups, e.g., a phenoxycarbonyl group; alkylamino groups, e.g., a methylamino group, an ethylamino group, a propylamino group, a butylamino group, and a dimethylamino group; cycloalkylamino groups, e.g., a cyclopropylamino group, a cyclobutylamino group, a cyclopentylamino group, a cyclohexylamino group, and a dicyclopropylamino group; alkylcarbamoyl groups, e.g., a methylcarbamoyl group, an ethylcarbamoyl group, a propylcarbamoyl group, a butylcarbamoyl group, and a dimethylcarbamoyl group; cycloalkylcarbamoyl groups, e.g., a cyclopropylcarbamoyl group, a cyclobutylcarbamoyl group, a cyclopentylcarbamoyl group, a cyclohexylcarbamoyl group, and a dicyclopropylcarbamoyl group; alkylcarbonylamino groups, e.g., a methylcarbonylamino group, an ethylcarbonylamino group, a propylcarbonylamino group, and a butylcarbonylamino group; cycloalkylcarbonylamino groups, e.g., a cyclopropylcarbonylamino group, a cyclobutylcarbonylamino group, a cyclopentylcarbonylamino group, and a cyclohexylcarbonylamino group; alkyloxycarbonylamino groups, e.g., a methyloxycarbonylamino group, an ethyloxycarbonylamino group, a propyloxycarbonylamino group, and a butyloxycarbonylamino group; cycloalkyloxycarbonylamino groups, e.g., a cyclopropyloxycarbonylamino group, a cyclobutyloxycarbonylamino group, a cyclopentyloxycarbonylamino group, and a cyclohexyloxycarbonylamino group; alkylthio groups, e.g., a methylthio group, an ethylthio group, a propylthio group, and a butylthio group; halogenated alkylthio groups, e.g., a trifluoromethylthio group, a difluoromethylthio group, and a trifluoroethylthio group; alkylsulfinyl groups, e.g., a methanesulfinyl group, an ethanesulfinyl group, a propanesulfinyl group, and a butanesulfinyl group; halogenated alkylsulfinyl groups, e.g., a trifluoromethanesulfinyl group, a difluoromethanesulfinyl group, and a trifluoroethanesulfinyl group; alkylsulfonyl groups, e.g., a methanesulfonyl group, an ethanesulfonyl group, a propanesulfonyl group, and a butanesulfonyl group; halogenated alkylsulfonyl groups, e.g., a trifluoromethanesulfonyl group, a difluoromethanesulfonyl group, and a trifluoroethanesulfonyl group; alkylsulfonamide groups, e.g., a methanesulfonamide group, an ethanesulfonamide group, a propanesulfonamide group, and a butanesulfonamide group; halogenated alkylsulfonamide groups, e.g., a trifluoromethanesulfonamide group, a difluoromethanesulfonamide group, and a trifluoroethanesulfonamide group; halogen atoms, e.g., a fluorine atom, a chlorine atom, a bromine atom, and an iodine atom; and acyl groups, e.g., an acetyl group and a benzoyl group.

In the compounds represented by Formula (3) and Formula (6), examples of groups which are represented by

X and which are to be eliminated can include halogen atoms typified by a chlorine atom, alkoxy groups typified by a methoxy group and an ethoxy group, aryloxy groups typified by a phenoxy group and a 4-nitrophenyl group, acyloxy groups typified by an acetyloxy group and a benzoyloxy group, alkoxycarbonyloxy groups typified by a methoxycarbonyloxy group, arylcarbonyloxy groups typified by a phenylcarbonyloxy group, alkylthio groups typified by a methylthio group, a 2,5-dioxopyrrolidinyloxy group, a benzotriazolyloxy group, and an imidazolyl group.

The compound represented by Formula (1) of the present invention is a new compound, and the compound represented by Formula (1) can be produced by a process described as Reaction formula (1).

Reaction formula (1)

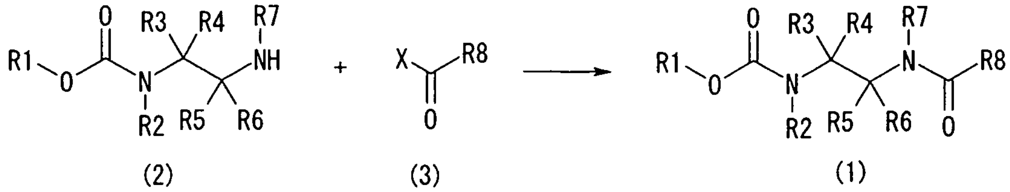

[In Reaction formula (1), R1, R2, R3, R4, R5, R6, and R7 represent the same substances as those in Formula (2), and R8 and X represent the same substances as those in Formula (3)]

In Reaction formula (1), a diamine derivative represented by Formula (2) or a salt thereof is reacted with a known carbonyl compound represented by Formula (3) in a solvent or without solvent in the presence of a base or without base and, thereby, a diamine derivative represented by Formula (1) can be produced.

Examples of bases used in the reaction represented by Reaction formula (1) can include alkali metal hydroxides, e.g., sodium hydroxide and potassium hydroxide; alkaline-earth metal hydroxide, e.g., magnesium hydroxide and calcium hydroxide; alkali metal hydrides, e.g., sodium hydride and potassium hydride; alkali metal alcoholates, e.g., sodium methoxide and sodium ethoxide; alkali metal oxides, e.g., sodium oxide; carbonates, e.g., potassium carbonate and sodium carbonate; phosphates, e.g., tripotassium phosphate, trisodium phosphate, dipotassium monohydrogen phosphate, and disodium monohydrogen phosphate; acetates, e.g., sodium acetate and potassium acetate; and organic bases, e.g., pyridine, 4-(dimethylamino)pyridine(DMAP), triethylamine, and 1,8-diazabicyclo[5.4.0]undec-7-ene(DBU).

The amounts of use of these bases are not specifically limited. When the above-described organic bases are used, these can also be used as solvents.

Examples of solvents used in the reaction represented by Reaction formula (1) can include water; halogenated hydrocarbons, e.g., dichloromethane and chloroform; aromatic hydrocarbons, e.g., benzene, toluene, and xylene; aliphatic hydrocarbons, e.g., hexane and heptane; aprotic polar solvents, e.g., dimethylformamide (DMF), dimethylacetamide (DMA), dimethyl sulfoxide (DMSO), 1,3-dimethyl-2-imidazolidinone (DMI), and 1-methyl-2-pyrrolidone (NMP); ethers, e.g., diethyl ether, diisopropyl ether, 1,2-dimethoxyethane, tetrahydrofuran (THF), and dioxane; and nitriles, e.g., acetonitrile and propionitrile, as long as the solvents do not react with compounds represented by, in particular, Formula (1), Formula (2), and Formula (3).

Preferably, the equivalent of the carbonyl compound represented by Formula (3) is 1 to 2 equivalent relative to the compound represented by Formula (2), and more preferably is 1 to 1.2 equivalent.

The reaction temperature and the reaction time of the above-described reaction can be varied within a wide range. In general, preferably, the reaction temperature is -20°C to 200°C, and more preferably is 0°C to 100°C. Preferably, the reaction time is 0.01 to 50 hours, and more preferably is 0.1 to 15 hours.

The amine derivative and a salt thereof represented by Formula (2) in Reaction Formula (1) other than commercially available compounds can be readily produced by known amine synthesis methods, e.g., the Gabriel method, the Delepine method, and reduction of a cyano group, amide, imine, oxime, and the like, and a method described in Tetrahedron Asymmetry, Vol. 11, page 1907 (2000).

The compound represented by Formula (3) in Reaction Formula (1) can be produced by a common method in which a known carboxylic acid derivative represented by Formula (4) is reacted with thionyl chloride, oxalyl chloride, phosgene, phosphorous oxychloride, phosphorous trichloride, phosphorous pentachloride, thionyl bromide, phosphorous tribromide, diethylaminosulfur trifluoride, 1,1'-carbonylbis-1H-imidazole, or the like.

The compound represented by Formula (3) in Reaction Formula (1) can also be produced by a common method in which a known carboxylic acid derivative represented by Formula (4) is reacted with alcohols, e.g., methyl alcohol or ethyl alcohol, or phenols, e.g., phenol or nitrophenol.

The compound represented by Formula (3) in Reaction Formula (1) can also be produced by a common method

in which a known carboxylic acid derivative represented by Formula (4) is reacted with chloroformates, e.g., methyl chloroformate or phenyl chloroformate.

The compound represented by Formula (3) in Reaction Formula (1) can also be produced by a common method in which a known carboxylic acid derivative represented by Formula (4) is reacted with N-hydroxysuccinic acid imide, 1-hydroxybenzotriazole, or the like.

The compound represented by Formula (1) of the present invention can also be produced by a method described as Reaction formula (2).

Reaction formula (2)

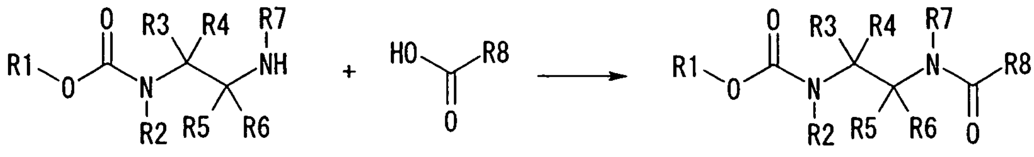

(2) (4) (1)

[In Reaction formula (2), R1, R2, R3, R4, R5, R6, and R7 represent the same substances as those in Formula (2) (Chemical formula 2), and R8 represents the same substance as that in Formula (4)]

In Reaction formula (2), a diamine derivative represented by Formula (2) or a salt thereof and a known carboxylic acid derivative represented by Formula (4) are condensed in a solvent or without solvent and, thereby, a diamine derivative represented by Formula (1) can be produced.

Examples of condensing agents in this case can include N,N'-dicyclohexylcarbodiimide, 1,1'-carbonylbis-1H-imidazole, 1-ethyl-3-(3-dimethylaminopropyl)carbodiimide hydrochloride, and 2-chloro-1,3-dimethylimidazolium chloride.

The amount of use of the condensing agent is 1 to 2 equivalent relative to the compound represented by Formula (4), and preferably is 1 to 1.2 equivalent.

In this case, an organic solvent similar to that used in the method represented by Reaction formula (1) can be used as long as the solvent do not react with compounds represented by, in particular, Formula (1), Formula (2), and Formula (4).

The amount of use of the carboxylic acid derivative represented by Formula (4) is 1 to 2 equivalent relative to the diamine derivative represented by Formula (2), and preferably is 1 to 1.2 equivalent.

The reaction temperature and the reaction time of the above-described reaction can be varied within a wide range. In general, the reaction temperature is -20°C to 200°C, and preferably is 0°C to 100°C. The reaction time is 0.01 to 50 hours, and preferably is 0.1 to 15 hours.

The compound represented by Formula (1) of the present invention can also be produced by a method described as Reaction formula (3).

Reaction formula (3)

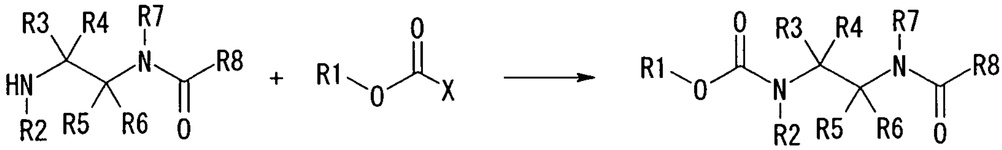

(5) (6) (1)

[In Reaction formula (3), R2, R3, R4, R5, R6, R7, and R8 represent the same substances as those in Formula (5), and R1 and X represent the same substances as those in Formula (6)]

In Reaction formula (3), a diamine derivative represented by Formula (5) or a salt thereof is reacted with a known compound represented by Formula (6) in a solvent or without solvent in the presence of a base or without base and, thereby, a diamine derivative represented by Formula (1) can be produced.

- The compound represented by Formula (6) in Reaction Formula (3) can be produced by a common method in which a corresponding alcohols are reacted with, for example, phosgenes, e.g., phosgene and triphosgene or chloroformates, e.g., phenylchloroformate.

In this case, bases similar to those used in the method represented by Reaction formula (1) can be used.
The amounts of use of these bases are not specifically limited. When the above-described organic bases are used, these can also be used as solvents.
In this case, an organic solvent similar to that used in the method represented by Reaction formula (1) can be used as long as the solvent do not react with compounds represented by, in particular, Formula (1), Formula (5), and Formula (6).
The amount of use of the compound represented by Formula (6) is 1 to 4 equivalent relative to the diamine derivative represented by Formula (5), and preferably is 1 to 2 equivalent.
The reaction temperature and the reaction time of the above-described reaction can be varied within a wide range. In general, the reaction temperature is -20°C to 200°C, and preferably is 0°C to 100°C. The reaction time is 0.01 to 50 hours, and preferably is 0.1 to 15 hours.
The diamine derivative represented by Formula (1) may include an asymmetric carbon atom depending on the types of substituents, and there may be optical isomers, diastereoisomers, racemates, and mixtures of arbitrary proportions. All types of these isomers and mixtures thereof are included in the present invention.
A plant disease control agent containing the diamine derivative represented by Formula (1), which is the compound of the present invention, as an active ingredient exerts an excellent effect of controlling the rice blast (Pyricularia oryzae) and the like.
The diamine derivative represented by Formula (1), which is the compound of the present invention, can be used as a mixture with other plant disease control agents; pesticides, e.g., insecticides, herbicides, and plant growth regulators; soil conditioners; or fertilizable materials, as a matter of course, and in addition, can be formulated together with them.
The compound of the present invention may be used alone, but preferably is put to use in the form of a composition prepared by mixing with a carrier including a solid or liquid diluent. Here, the carrier refers to a synthesized or natural, inorganic or organic substance blended in order to accelerate an active ingredient to reach sites to be treated or to smooth the way to store, transport, and handle the active ingredient.
Examples of appropriate solid carriers include clay, e.g., montmorillonite, kaolinite, and bentonite; inorganic materials, e.g., diatomaceous earth, kaolin, talc, vermiculite, gypsum, calcium carbonate, amorphous silicon dioxide, and ammonium sulfate; vegetable organic materials, e.g., soybean meal, and wheat flour; and urea.
Examples of appropriate liquid carriers include aromatic hydrocarbons, e.g., toluene, xylene, and cumene; paraffin hydrocarbons, e.g., kerosene and mineral oil, halogenated hydrocarbons, e.g., carbon tetrachloride, chloroform, and dichloroethane; ketones, e.g., acetone and methyl ethyl ketone; ethers, e.g., dioxane, tetrahydrofuran, and diethylene glycol dimethyl ether; alcohols, e.g., methanol, ethanol, propanol, and ethylene glycol; aprotic polar solvents, e.g., dimethylformamide, dimethyl sulfoxide, and 1-methyl-2-pyrrolidone; and water.
Furthermore, in order to enhance the effect of the compound of the present invention, the following inert ingredients can also be used alone or in combination according to purposes in consideration of types of formulation, situations of application, and the like.
Examples of inert ingredients for the purposes of emulsification, dispersion, spread, wetting, bonding, stabilization, and the like include anionic surfactants, e.g., lignin sulfonates, alkylbenzene sulfonates, alkylsulfuric acid ester salts, polyoxyalkylene alkyl sulfates, and polyoxyalkylene alkylphosphoric acid ester salts; nonionic surfactants, e.g., polyoxyalkylene alkyl ethers, polyoxyalkylene alkylaryl ethers, polyoxyalkylene alkylamines, polyoxyalkylene alkylamides, polyoxyalkylene alkylthioethers, polyoxyalkylene aliphatic acid esters, glycerin aliphatic acid esters, sorbitan aliphatic acid esters, polyoxyalkylene sorbitan aliphatic acid esters, and polyoxypropylene polyoxyethylene block polymers; lubricants, e.g., calcium stearate and wax; stabilizers, e.g., isopropylhydrodiene phosphate; and furthermore, methyl cellulose, carboxymethyl cellulose, casein, and gum arabic. However, these components are not limited to the above-described substances.
In general, the amount of the active ingredient in the compound of the present invention is 0.5 to 20 percent by weight with respect to a dust, 5 to 50 percent by weight with respect to an emulsifable concentrate, 10 to 90 percent by weight with respect to a wettable powder, 0.1 to 20 percent by weight with respect to a granule, and 10 to 90 percent by weight with respect to a flowable formulation. On the other hand, in general, the amount of the carrier in each type of formulation is 60 to 99 percent by weight with respect to a dust, 40 to 95 percent by weight with respect to an emulsifable concentrate, 10 to 90 percent by weight with respect to a wettable powder, 80 to 99 percent by weight with respect to a granule, and 10 to 90 percent by weight with respect to a flowable formulation. In general, the amount of the inert ingredient is 0.1 to 20 percent by weight with respect to a dust, 1 to 20 percent by weight with respect to an emulsifable concentrate, 0.1 to 20 percent by weight with respect to a wettable powder, 0.1 to 20 percent by weight with respect to

a granule, and 0.1 to 20 percent by weight with respect to a flowable formulation.

The present invention will be described below in further detail with reference to examples and test examples.

[Example 1]

Process for synthesizing N-(chloromethyloxycarbonyl)-N'-(4-methylbenzoyl)-3-methyl-1,2-butanediamine (Compound No. 1)

To a mixture(6ml) of 0.50 g of N-(4-methylbenzoyl)-3-methyl-1,2-butanediamine hydrochloride in dichloromethane was added 0.39 g of triethylamine under an ice-cooled condition. And then, a solution (2ml) of 0.25 g of chloromethyl chloroformate in dichloromethane was added under an ice-cooled condition, and the mixture was stirred for 4 hours at room temperature. The mixture was -purified by column chromatography on silica gel (ethyl acetate/hexane), so that 0.43 g of the -title compound was prepared as white crystals.

[Example 2]

Process for synthesizing N-(2,2,2-trifluoroethyloxycarbonyl)-N'-(4-methylbenzoyl)-3-methyl-1,2-butanediamine (Compound No. 6)

Under an ice-cooled condition, a solution (6ml) of 0.78 g of 4-nitrophenyl chloroformate in dichloromethane was added to a mixed solution of 0.42 g of 2,2,2-trifluoroethanol and 0.43 g of triethylamine. After the reaction solution was stirred for 1 hour under an ice-cooled condition, 0.50 g of N-(4-methylbenzoyl)-3-methyl-1,2-butanediamine hydrochloride was added under an ice-cooled condition. After 0.21 g of triethylamine was added under an ice-cooled condition, the reaction solution was stirred for 1 hour under an ice-cooled condition and for 2 hours at room temperature. The reaction solution was stood for one night at room temperature and, subsequently, was concentrated under a reduced pressure. 50ml of ethyl acetate was added to the residue and the resulting mixture was washed with water. The organic layer was dried over anhydrous magnesium sulfate, and was concentrated under a reduced pressure so as to produce a solid. The resulting solid was washed with diisopropyl ether and, subsequently, was purified by column chromatography on silica gel (ethyl acetate/hexane), so that 0.34 g of the title compound was prepared as white crystals.

[Example 3]

Process for synthesizing N-(2,2,2-trichloroethyloxycarbonyl)-N'-(4-methylbenzoyl)-3-methyl-1,2-butanediamine (Compound No. 7)

To a mixture(5ml) of 0.50 g of N-(4-methylbenzoyl)-3-methyl-1,2-butanediamine hydrochloride in dichloromethane was added 0.43 g of triethylamine under an ice-cooled condition. And then, 0.41 g of 2,2,2-trichloroethyl chloroformate was added under an ice-cooled condition, and the mixture was stirred for 4 hours at room temperature. The mixture was purified by column chromatography on silica gel (ethyl acetate/hexane), so that 0.52 g of the title compound was prepared as white crystals.

[Example 4]

Process for synthesizing N-(1,1,1,3,3,3-hexafluoro-2-propyloxycarbonyl)-N'-(4-methylbenzoyl)-3-methyl-1,2-butanediamine (Compound No. 8)

Under an ice-cooled condition, a mixed solution of 0.65 g of 1,1,1,3,3,3-hexafluoro-2-propanol and 0.43 g of triethylamine was added to a solution (6ml) of 0.78 g of 4-nitrophenyl chloroformate in dichloromethane. After the reaction solution was stirred for 1 hour under an ice-cooled condition, 0.50 g of N-(4-methylbenzoyl)-3-methyl-1,2-butanediamine hydrochloride was added under an ice-cooled condition. After the reaction solution was stirred for 15 minutes under an ice-cooled condition, 0.21 g of triethylamine was added under an ice-cooled condition. The reaction solution was stirred for 1 hour under an ice-cooled condition and for 1 hour at room temperature and, subsequently, was stood for one night. 50ml of dichloromethane was added to the solution and the solution was washed with water. The organic layer was dried over anhydrous magnesium sulfate and, subsequently, was concentrated under a reduced pressure. The resulting solid was purified by column chromatography on silica gel (ethyl acetate/hexane), so that 0.38 g of the title compound was prepared as white crystals.

[Example 5]

Process for synthesizing of N-(2-chlorocyclohexyloxycarbonyl)-N'-(4-methylbenzoyl)-3-methyl-1,2-butanediamine (Compound No. 17)

Under an ice-cooled condition, a solution (25ml) of 5.00 g of 4-nitrophenyl chloroformate in dichloromethane was added to a solution (50ml) of 1.96 g of pyridine and 3.33 g of 2-chlorocyclohexanol in dichloromethane. After the reaction solution was stirred for 3.5 hours at room temperature, 100 ml of dichloromethane was added, and washed with a saturated sodium hydrogencarbonate aqueous solution. The organic layer was dried over anhydrous magnesium sulfate and, subsequently, was concentrated under a reduced pressure. The resulting oil was purified by column chromatography on silica gel (dichloromethane/hexane), so that 4.43 g of (2-chlorocyclohexyl)-(4-nitrophenyl)carbonate was prepared as a yellow oil.

Under an ice-cooled condition, 2.00 g of N-(4-methylbenzoyl)-3-methyl-1,2-butanediamine hydrochloride was added to a solution (50ml) of 4.43 g of (2-chlorocyclohexyl)-(4-nitrophenyl)carbonate in tetrahydrofuran. After 0.78 g of triethylamine was added under an ice-cooled condition, the reaction solution was stirred for 1 hour under an ice-cooled condition and for 2 hours at room temperature. The reaction solution was stood for one night at room temperature and, subsequently, was concentrated under a reduced pressure. 200ml of ethyl acetate was added to the residue and the resulting mixture was washed with water. The organic layer was dried over anhydrous magnesium sulfate and, subsequently, was concentrated under a reduced pressure. The resulting oil was purified by column chromatography on silica gel (ethyl acetate/hexane), so that 1.38 g of the title compound was prepared as white crystals.

Compounds represented by Formula (1) which can be produced as in Examples 1 to 5 are shown in Table 1 (Table 1-1 to Table 1-3) below. The values of some properties thereof are shown in Table 2 (Table 2-1 to Table 2-9). In Table 1, Me represents a methyl group, Et represents an ethyl group, n-Pr represents a normal propyl group, i-Pr represents an isopropyl group, and cyclohexyl represents a cyclohexyl group.

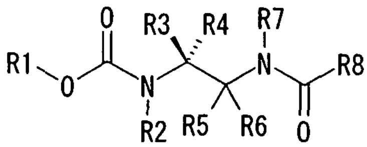

[1]

Table 1-1 Compound (1) represented by Formula (1)

| Compound No. | R1 | R2 | R3 | R4 | R5 | R6 | R7 | R8 |
|---|---|---|---|---|---|---|---|---|
| 1 | ClCH2 | H | i-Pr | H | H | H | H | 4-MeC6H4 |
| 2 | FCH2CH2 | H | i-Pr | H | H | H | H | 4-MeC6H4 |
| 3 | ClCH2CH2 | H | i-Pr | H | H | H | H | 4-MeC6H4 |
| 4 | BrCH2CH2 | H | i-Pr | H | H | H | H | 4-MeC6H4 |
| 5 | ICH2CH2 | H | i-Pr | H | H | H | H | 4-MeC6H4 |
| 6 | F3CCH2 | H | i-Pr | H | H | H | H | 4-MeC6H4 |
| 7 | Cl3CCH2 | H | i-Pr | H | H | H | H | 4-MeC6H4 |
| 8 | F3C(F3C)CH | H | i-Pr | H | H | H | H | 4-MeC6H4 |
| 9 | FCH2(FCH2)CH | H | i-Pr | H | H | H | H | 4-MeC6H4 |
| 10 | ClCH2CH2CH2 | H | i-Pr | H | H | H | H | 4-MeC6H4 |
| 11 | BrCH2(BrCH2)CH | H | i-Pr | H | H | H | H | 4-MeC6H4 |
| 12 | F3C(BrCH2)CH | H | i-Pr | H | H | H | H | 4-MeC6H4 |
| 13 | F3CCF2CF2CH2 | H | i-Pr | H | H | H | H | 4-MeC6H4 |

(continued)

| Compound No. | R1 | R2 | R3 | R4 | R5 | R6 | R7 | R8 |
|---|---|---|---|---|---|---|---|---|
| 14 | F3CCF2CF2CF2CH 2CH2 | H | i-Pr | H | H | H | H | 4-MeC6H4 |
| 15 | CH2=C(Cl)CH2 | H | i-Pr | H | H | H | H | 4-MeC6H4 |
| 16 | 2-fluoro-cyclohexyl | H | i-Pr | H | H | H | H | 4-MeC6H4 |

Table 1-2 Compound (1) represented by Formula (1)

| | | | | | | | | |
|---|---|---|---|---|---|---|---|---|
| 17 | 2-chloro-cyclohexyl | H | i-Pr | H | H | H | H | 4-MeC6H4 |
| 18 | 2-iodo-cyclohexyl | H | i-Pr | H | H | H | H | 4-MeC6H4 |
| 19 | EtO(F3C)CH | H | i-Pr | H | H | H | H | 4-MeC6H4 |
| 20 | F3CCH2 | H | H | H | H | H | H | 4-MeC6H4 |
| 21 | F3CCH2 | H | Et | H | H | H | H | 4-MeC6H4 |
| 22 | F3CCH2 | H | n-Pr | H | H | H | H | 4-MeC6H4 |
| 23 | F3CCH2 | H | i-Bu | H | H | H | H | 4-MeC6H4 |
| 24 | F3CCH2 | H | t-Bu | H | H | H | H | 4-MeC6H4 |
| 25 | F3CCH2 | H | 2-butyl | H | H | H | H | 4-MeC6H4 |
| 28 | F3CCH2 | H | i-Pr | H | H | H | H | 2-benzofuranyl |
| 30 | F3C(CH3)CH | H | i-Pr | H | H | H | H | 4-MeC6H4 |

Table 1-3 Compound (1) represented by Formula (1)

| | | | | | | | | |
|---|---|---|---|---|---|---|---|---|
| 31 | F3CCH2 | H | t-Bu | H | H | H | H | 2-benzofuranyl |
| 32 | F3CCH2 | H | 2-butyl | H | H | H | H | 2-benzofuranyl |
| 33 | F3CCH2 | H | 2-butyl | H | H | H | H | 2-benzothiazolyl |
| 34 | F3CCH2 | H | i-Pr | H | H | H | H | 2-benzothiophenyl |
| 35 | F3CCH2 | H | t-Bu, H | | H | H | H | 4-MeC6H4 |
| 36 | F3CCH2 | H | cyclopentyl | H | H | H | H | 4-MeC6H4 |
| 37 | F3CCH2 | H | i-Pr | H | H | H | H | 6-fluoro-2-benzothiazolyl |
| 38 | F3CCH2 | H | i-Pr | H | H | H | H | 2-benzothiazolyl |
| 39 | F3CCH2 | H | 3- pentyl | H | H | H | H | 4-MeC6H4 |
| 40 | F3CCH2 | H | Me, H | | H | H | H | 2-benzofuranyl |

Table 2-1 Property value of Compound (1)

| Compound No. | Property value |
|---|---|
| 1 | $^1$H NMR ($CDCl_3$, ppm): 1.00 (3H, d, J=7.3 Hz), 1.02 (3H, d, J=7.1 Hz), 1.85-1.94 (1H, m), 2.39 (3H, s), 3.45-3.51 (1H, m), 3.63-3.76 (2H, m), 5.24 (1H, d, J=8.5 Hz), 5.66 (1H, d, J=6.1 Hz), 5.72 (1H, d, J= 6.1 Hz), 6.59 (1H, br-s), 7.22 (2H, d, J=7.8 Hz), 7.66 (2H, d, J=8.1 Hz). |

(continued)

| Compound No. | Property value | |
|---|---|---|
| 2 | $^1H$ NMR ($CDCl_3$, ppm): | 1.00 (3H, d, J=7.3 Hz), 1.02 (3H, d, J=6.8 Hz), 1.88-1.89 (1 H, m), 2.39 (3H, s), 3.52-3.72 (2H, m), 3.69-3.72 (1 H, m), 4.23-4.25 (1 H, m), 4.30-4.45 (1 H, m), 4.44-4.80 (1 H, m), 4.56-4.60 (1 H, m), 4.97 (1 H, br-d, J=8.8 Hz), 6.78 (1 H, br-s), 7.23 (2H, d, J=8.0 Hz), 7.67 (2H, d, J=8.3Hz). |
| 3 | $^1H$ NMR ($CDCl_3$, ppm): | 0.97 (3H, d, J=6.3 Hz), 0.99 (3H, d, J=6.3 Hz), 1.83-1.88 (1H, m), 2.38 (3H, s), 3.43-3.71 (5H, m), 4.19-4.24 (2H, m), 5.38 (1 H, br-d, J=6.8 Hz), 7.06 (1 H, br-s), 7.17 (2H, d, J=7.8 Hz), 7.66 (2H, d, J=7.8 Hz). |
| 4 | $^1H$ NMR ($CDCl_3$, ppm): | 1.00 (3H, d, J=6.8 Hz), 1.02 (3H, d, J=6.9 Hz), 1.87-1.89 (1H, m), 2.40 (3H, s), 3.38-3.41 (2H, m), 3.51-3.71 (3H, m), 4.25-4.33 (2H, m), 4.98 (1H, br-d, J=7.8Hz), 6.75 (1H, br-s), 7.23 (2H, d, J=7.8 Hz), 7.67 (2H, d, J=8.3 Hz). |

Table 2-2 Property value of Compound (2)

| | | |
|---|---|---|
| 5 | $^1H$ NMR ($CDCl_3$, ppm): | 1.00 (3H, d, J=7.6 Hz), 1.02 (3H, d, J=6.8 Hz), 1.87-2.05 (1H, m), 2.41 (3H, s), 3.13-3.17 (2H, m), 3.46-3.50 (1H, m), 3.59-3.70 (2H, m), 4.22-4.28 (2H, m), 4.93 (1 H, br-d, J=8.8 Hz), 6.73 (1H, br-s), 7.23 (2H, d, J=7.8 Hz), 7.67 (2H, d, J=8.3 Hz). |
| 6 | $^1H$ NMR ($CDCl_3$, ppm): | 0.96-1.03 (6H, m), 1.85-1.91 (1 H, m), 2.39(3H, s), 3.46-3.51 (1H, m), 3.61-3.72 (2H, m), 4.35-4.46 (2H, m), 5.26 (1 H, d, J=8.3 Hz), 6.62 (1 H, br-s), 7.21-7.23 (2H, m), 7.63-7.65 (2H, m) |
| 7 | $^1H$ NMR ($CDCl_3$, ppm): | 1.02 (3H, d, J=5.9 Hz), 1.03 (3H, d, J=6.6 Hz), 1.86-1.94 (1H, m), 2.39 (3H, s), 3.48-3.53 (1H, m), 3.63-3.77 (2H, m), 4.64 (1H, d, J=12.0 Hz), 4.73 (1H, d, J=12.0 Hz), 5.25 (1 H, d, J=8.3 Hz), 6.64 (1 H, br-s), 7.21 (2H, d, J=7.8 Hz), 7.64 (2H, d, J=8.1 Hz). |
| 8 | $^1H$ NMR ($CDCl_3$, ppm): | 1.01 (3H, d, J=6.8 Hz), 1.02 (3H, d, J=7.1 Hz), 1.86-1.94 (1H, m), 2.39 (3H, s), 3.46-3.52 (1 H, m), 3.54-3.76 (2H, m), 5.56-5.67 (2H, m), 6.42 (1H, br-s), 7.22(2H, d, J=7.8 Hz), 7.70 (2H, J=8.1 Hz). |

Table 2-3 Property value of Compound (3)

| | | |
|---|---|---|
| 9 | $^1H$ NMR ($CDCl_3$, ppm): | 1.00 (3H, d, J=6.8 Hz), 1.02 (3H, d, J=6.8 Hz), 1.84-1.92 (1 H, m), 2.39 (3H, s), 3.47-3.52 (1H, m), 3.59-3.74 (2H, m), 4.34-4.36 (1 H, m), 4.51-4.52 (1 H, m), 4.62-4.64 (1 H, m), 5.00-5.12 (2H, m), 6.69 (1 H, br-s), 7.22 (2H, d, J=8.1 Hz), 7.66 (2H, d, J=8.1 Hz). |
| 10 | $^1H$ NMR ($CDCl_3$, ppm): | 0.99 (3H, d, J=6.8 Hz), 1.01 (3H, d, J=6.8 Hz), 1.82-1.92 (1 H, m), 2.39 (3H, s), 3.43-3.57 (3H, m), 3.60-3.73 (2H,m), 4.10-4.20 (2H, m), 4.92 (1H, d, J=8.3 Hz), 6.82 (1H, br-s), 7.22 (2H, d, J=8.3 Hz), 7.67 (2H, d, J=8.1 Hz). |
| 11 | $^1H$ NMR ($CDCl_3$, ppm): | 1.01 (3H, d, J=6.8 Hz), 1.02 (3H, d, J=6.8 Hz), 1.85-1.90 (1H, m), 2.39 (3H, s), 3.34-3.74 (7H, m), 4.97-5.04 (2H, m), 6.62 (1H, br-s), 7.23 (2H, d, J=7.8 Hz), 7.66 (2H, dd, J=8.3 Hz, 2.2 Hz). |
| 12 | $^1H$ NMR ($CDCl_3$, ppm): | 1.00-1.04 (6H, m), 1.87-1.93 (1H, m), 2.39 (3H, s), 3.31-3.34 (1H, m), 3.43-3.52 (2H, m), 3.58-3.75(2H, m), 5.25-5.32 (1H, m), 5.41-5.47 (1H, m), 6.54-7.00 (1H, m), 7.21(2H, d, J=8.1 Hz), 7.62 (2H x 1/2, d, J=8.1 Hz), 7.66(2H x 1/2, d, J=8.3Hz). |

Table 2-4 Property value of Compound (4)

| | | |
|---|---|---|
| 13 | $^1H$ NMR ($CDCl_3$, ppm): | 0.99 (3H, d, J=7.1Hz), 1.01 (3H, d, J=7.1Hz), 1.84-1.92 (1H, m), 2.39 (3H, s), 3.46-3.53 (1 H, m), 3.61-3.73 (2H, m), 4.46-4.61 (2H, m), 5.21 (1 H, br-d, J=7.6Hz), 6.55 (1 H, br-s), 7.21 (2H, dd, J=8.1Hz, 0.5Hz), 7.63 (2H, d, J=8.1 Hz). |
| 14 | $^1H$ NMR ($CDCl_3$, ppm): | 0.99 (3H, d, J=7.6Hz), 1.01 (3H, d, J=7.1Hz), 1.82-1.89 (1H, m), 2.21-2.40 (5H, m), 3.43-3.47(1 H, m), 3.60-3.69 (2H, m), 4.26-4.31 (2H, m), 4.95 (1 H, br-d, J=8.5Hz), 6.67 (1H, br-s), 7.22 (2H, d, J=7.8Hz), 7.66 (2H, d, J=8.3Hz). |
| 15 | $^1H$ NMR ($CDCl_3$, ppm): | 1.00 (3H, d, J=7.1 Hz),1.02 (3H, d, J=6.8 Hz), 1.84-1.92 (1H, m), 2.39 (3H, s), 3.45-3.50 (1 H, m), 3.60-3.75 (2H, m), 4.56 (1H, d, J=13.9 Hz), 4.62 (1H, d, J=13.9 Hz), 5.06 (1 H, d, J=8.3 Hz), 5.26 (1 H, s), 5.34 (1 H, d, J=1.5 Hz), 6.73 (1 H, br-d, J=3.2Hz), 7.22 (2H, d, J=8.1 Hz), 7.65 (1 H, d, J=8.3 Hz). |
| 16 | $^1H$ NMR ($CDCl_3$, ppm): | 0.99-1.02 (6H, m), 1.21-1.35 (3H, m), 1.71-1.90 (4H, m), 2.05-2.10 (2H, m), 2.38 (3H, s), 3.44-3.71 (3H, m), 4.26-4.40 (1H, m), 4.67-4.85 (2H, m), 6.84 (1H, br-s), 7.21 (2H, d, J=7.1 Hz), 7.65-7.69 (2H, m). |

Table 2-5 Property value of Compound (5)

| | | |
|---|---|---|
| 17 | $^1H$ NMR ($CDCl_3$, ppm): | 0.99-1.03 (6H, m), 1.24-1.39 (3H, m), 1.59-1.71 (3H, m), 1.84-1.88 (1H, m), 2.05-2.17 (2H, m), 2.38 (3H, d, J=1.5 Hz), 3.49-3.74 (4H, m), 4.62-4.74 (1 H, m), 4.88 (1H, d, J=5.6Hz), 6.80 (1H, br-s), 7.20-7.22 (2H, m), 7.67 (2H, d, J=7.1 Hz). |
| 18 | $^1H$ NMR ($CDCl_3$, ppm): | 0.99-1.05 (6H, m), 1.19-2.38 (12H, m), 3.46-3.78 (3H, m), 3.89-4.04 (1H, m), 4.71-4.87 (2H, m), 6.79 (1H, br-s), 7.21 (2H, d, J=8.3 Hz), 7.66-7.70 (2H, m). |
| 19 | $^1H$ NMR ($CDCl_3$, ppm): | 0.96-1.03 (6H, m), 1.26 (3H, t, J= 7.1Hz), 1.8-1.9 (1 H, m), 2.39 (3H, s), 3.47-3.72 (5H, m), 5.26-5.35 (1H, m), 5.89-5.91 (1H, m), 6.5-6.6 (1H, m), 7.20-7.22 (2H, m), 7.60-7.67 (2H, m). |
| 20 | $^1H$ NMR ($CDCl_3$, ppm): | 2.40 (3H, s), 3.46-3.50 (2H, m), 3.60-3.64 (2H, m), 4.43 (2H, q, J = 8.5 Hz), 5.57 (1H, br-s), 6.66 (1 H, br-s), 7.23-7.26 (2H, m), 7.66-7.68 (2H, m). |
| 21 | $^1H$ NMR ($CDCl_3$, ppm): | 1.01 (3H, t, J=7.6 Hz), 1.50-1.71 (2H, m), 2.39 (3H, s), 3.49-3.62 (2H, m), 3.72-3.81 (1H, m), 4.34-4.50 (2H, m), 5.29 (1H, d, J=8.1 Hz), 6.66 (1H, br-s) 7.22-7.24 (2H, m), 7.65 (2H, d, J=8.3 Hz). |

Table 2-6 Property value of Compound (6)

| | | |
|---|---|---|
| 22 | $^1H$ NMR ($CDCl_3$, ppm): | 0.95 (3H, t, J=7.2 Hz), 1.37-1.58 (4H, m), 2.40 (3H, s), 3.48-3.61 (2H, m), 3.82-3.87 (1H, m), 4.36-4.47 (2H, m), 5.25 (1H, d, J=8.5 Hz), 6.68 (1H, br-s), 7.23 (2H, d, J=7.8 Hz), 7.65 (2H, d, J=8.3 Hz). |
| 23 | $^1H$ NMR ($CDCl_3$, ppm): | 0.95 (3H, d, J=6.3 Hz), 0.96 (3H, d, J=6.6 Hz), 1.36-1.47 (2H, m), 1.69-1.74 (1 H, m), 2.39 (3H, s), 3.48-3.55 (2H, m), 3.90-3.95 (1 H, m), 4.36-4.46(2H, m), 5.11 (1H, d, J=8.5 Hz), 6.65 (1 H, br-s), 7.23 (2H, d, J=8.3 Hz), 7.65 (2H, d, J=8.1 Hz). |
| 24 | $^1H$ NMR ($CDCl_3$, ppm): | 1.04 (9H, s), 2.38 (3H, s), 3.50-3.53 (1 H, m), 3.65-3.69(2H, m), 4.31-4.42 (2H, m), 5.21 (1 H, br-d, J=8.8 Hz), 6.55 (1H, br-s), 7.19 (2H, d, J=8.1 Hz), 7.60-7.62 (2H, m). |

(continued)

| | | |
|---|---|---|
| 25 | $^{1}H$ NMR ($CDCl_3$, ppm): | 0.95(3H, t, J=7.3 Hz), 0.99 (3H, d, J= 6.8 Hz), 1.20-1.25 (1 H, m), 1.53-1.67 (2H, m), 2.39 (3H, s), 3.46-3.51 (1H, m), 3.61-3.69 (1H, m), 3.73-3.78 (1 H, m), 4.35-4.45 (2H, m), 5.25 (1 H, d, J= 8.5 Hz), 6.60 (1 H, br-s), 7.21-7.24 (2H, m), 7.64 (2H, d, J= 8.3Hz). |

Table 2-7 Property value of Compound (7)

| | | |
|---|---|---|
| 28 | $^{1}H$ NMR ($CDCl_3$, ppm): | 1.01 (3H, d, J=7.1 Hz), 1.03 (3H, d, J=6.8 Hz), 1.88-1.93 (1H, m), 3.54-3.74 (3H, m), 4.36-4.48 (2H, m), 5.24 (1H, d, J=8.8 Hz), 6.95 (1H, br-s), 7.26-7.31 (1H, m), 7.39-7.50 (3H, m), 7.66 (1H, d, J=8.1 Hz) |
| 30 | $^{1}H$ NMR ($CDCl_3$, ppm): | 0.98 (3H, d, J=6.8Hz), 1.00 (3H, d, J=6.8Hz), 1.18 (1/2H, d, J=6.8Hz), 1.36 (1/2H, d, J=6.8Hz), 1.84-1.86 (1H, m), 3.42-3.66 (2H, m) 3.67-3.70 (1H, m), 5.03-5.10 (1H, m), 5.22 (1H, br-s), 5.12-5.19 (1H, m), 6.58 (1 H, br-s), 7.18-7.22 (2H, m), 7.60-7.65 (2H, m). |

Table 2-8 Property value of Compound (8)

| | | |
|---|---|---|
| 31 | $^{1}H$ NMR ($CDCl_3$, ppm): | 0.97 (9H, s), 3.54-3.58 (1H, s), 3.61-3.70 (2H, m), 4.31-4.41 (2H, m), 5.18 (1 H, br-s), 6.91 (1 H, br-s), 7.23-7.27 (1 H, m), 7.35-7.45 (1H, d, J=7.8Hz). |
| 32 | $^{1}H$ NMR ($CDCl_3$, ppm): | 0.92-1.00 (6H, m), 1.55-1.58 (1H, m), 3.54-3.54 (1H, m), 3.76-3.79 (1H, m), 4.37-4.42 (2H, m), 5.21 (1H, br-d, J=8.9Hz), 6.92 (1H, br-s), 7.26-7.38 (1 H, m), 7.40-7.49 (3H, m), 7.65 (1 H, d, J=7.6Hz). |
| 33 | $^{1}H$ NMR ($CDCl_3$, ppm): | 0.98-1.00 (3H, m), 0.92-0.96 (3H, m), 1.22-1.26 (2H, m), 1.60-1.70 (2H, m), 3.60-3.65 (2H, m), 3.66-3.70 (1H, m), 4.35-4.42 (1 H, m), 7.46-7.56 (2H, m), 7.60-7.63 (1H, m), 7.96 (1H, d, J=7.6Hz), 8.06 (1H, d, J=8.4Hz). |
| 34 | $^{1}H$ NMR ($CDCl_3$, ppm): | 1.02 (6H, t, J=6.8Hz), 1.87-1.95 (1H, m), 3.53-3.76 (3H, m), 4.36-4.52 (2H, m), 5.20 (1H, d, J=8.8Hz), 6.78 (1 H, br-s), 7.37-7.45 (2H, m), 7.73 (1H, s), 7.82-7.86 (2H, m). |
| 35 | $^{1}H$ NMR ($CDCl_3$, ppm): | 1.01 (9H, s), 2.39 (3H, s), 3.50-3.54 (1 H, m), 3.61-3.69 (2H, m), 4.34-4.42 (2H, m), 5.12 (1 H, d, J=8.8Hz), 6.48 (1 H, br), 7.22 (2H, d, J=8.3Hz), 7.62 (2H, d, J=8.3Hz). |
| 36 | $^{1}H$ NMR ($CDCl_3$, ppm): | 1.27-1.36 (2H, m), 1.53-1.71 (4H, m), 1.79-1.86 (2H, m), 1.95-2.01 (1 H, m), 2.39 (3H, s), 3.52-3.63 (2H, m), 3.68-3.74 (1 H, m), 4.35-4.46 (2H, m), 5.22 (1 H, d, J=8.3Hz), 6.68 (1H, br), 7.22 (2H, d, J=8.3Hz), 7.64 (2H, d, J=8.3Hz). |

Table 2-9 Property value of Compound (9)

| | | |
|---|---|---|
| 37 | $^{1}H$ NMR ($CDCl_3$, ppm): | 1.01 (3H, d, J=6.8Hz), 1.04 (3H, d, J=6.8Hz), 1.86-1.95 (1H, m), 3.61-3.79 (3H, m), 4.34-4.49 (2H, m), 5.15 (1H, d, J=8.8Hz), 7.23-7.32 (1 H, m), 7.63-7.65 (2H, m), 8.00-8.04 (1H, m). |
| 38 | $^{1}H$ NMR ($CDCl_3$, ppm): | 1.01 (3H, d, J=6.8Hz), 1.04 (3H, d, J=6.8Hz), 1.84-1.94 (1H, m), 3.59-3.79 (3H, m), 4.34-4.49 (2H, m), 5.24 (1 H, d, J=9.3Hz), 7.48-7.58 (2H, m), 7.74 (1H, br-s), 7.97 (1H, d, J=7.3Hz), 8.07 (1H, d, J=7.8Hz). |
| 39 | $^{1}H$ NMR ($CDCl_3$, ppm): | 0.88-0.99 (6H, m), 1.29-1.51 (5H, m), 3.49-3.56 (1H, m), 3.64-3.72 (1 H, m), 3.95-4.01 (1 H, m), 4.36-4.46 (2H, m), 5.15 (1 H, d, J=9.3), 6.99 (1 H, br), 7.27-7.31 (1H, m), 7.39-7.51 (3H, m), 7.67 (1H, d, J=7.8Hz). |

(continued)

| | | |
|---|---|---|
| 40 | $^1$H NMR ($CDCl_3$, ppm): | 1.26 (3H, d, J= 6.8Hz), 3.52-3.62 (1 H, m), 3.93-3.99 (1 H, m), 4.35-4.49 (2H, m), 5.44 (1 H, d, J=6.8Hz), 7.01 (1 H, br-s), 7.26-7.30 (1H, m), 7.39-7.43 (1H, m), 7.46-7.50 (1H, m), 7.66 (1H, d, J=7.8Hz). |

Formulation examples and Test examples

The formulation examples of plant disease control agents according to the present invention and the plant disease control activity test examples will be described below. In the following description, "part" refers to "part by weight".

[Example 6] Granule

Thirty parts of the compound (1) of the present invention, 22 parts of bentonite, 45 parts of talc, and 3 parts of Sorpol 5060 (surfactant: TOHO Chemical Industry Co., Ltd., trade name) were kneaded homogeneously, and were granulated with a basket granulator. Subsequently, drying was performed, so that 100 parts of granules were prepared.

[Example 7] Granule

After 15 parts of the compound (2) of the present invention, 60 parts of bentonite, 21 parts of talc, 1 part of sodium dodecylbenzenesulfonate, 1 part of polyoxyethylene alkyl aryl ether, and 2 parts of sodium ligninsulfonate were mixed, an appropriate amount of water was added. The resulting mixture was kneaded homogeneously, and was granulated with a basket granulator. Subsequently, drying was performed, so that 100 parts of granules were prepared.

[Example 8] Wettable powder

Fifty parts of the compound (3) of the present invention, 40 parts of calcium carbonate, 5 parts of Sorpol 5039 (mixture of anionic surfactant and amorphous silicon dioxide: TOHO Chemical Industry Co., Ltd., trade name), and 5 parts of amorphous silicon dioxide were mixed and pulverized homogeneously, so that a wettable powder was prepared.

[Example 9] Wettable powder

Thirty parts of the compound (4) of the present invention, 63 parts of kaolinite, 5 parts of Sorpol 5039 (mixture of anionic surfactant and amorphous silicon dioxide: TOHO Chemical Industry Co., Ltd., trade name), and 2 parts of amorphous silicon dioxide were mixed and pulverized homogeneously, so that a wettable powder was prepared.

[Example 10] Emulsifable concentrate

Twenty parts of the compound (5) of the present invention, 55 parts of xylene, 20 parts of N,N-dimethylformamide, and 5 parts of Sorpol 2680 (surfactant) were mixed homogeneously, so that an emulsifable concentrate was prepared.

[Example 11] Suspension concentrate

Components other than the active ingredients among 40 parts of the compound (6) of the present invention, 5 parts of Sorpol 3353 (nonionic surfactant: TOHO Chemical Industry Co., Ltd., trade name), 5 parts of 1% aqueous solution of xanthan gum, 40 parts of water, and 10 parts of ethylene glycol were dissolved homogeneously, the compound of the present invention was added, and agitation was performed adequately. Subsequently, wet grinding was performed with a sand mill, so that a suspension concentrate was prepared.

[Example 12] Dust formulation

Five parts of the compound (7) of the present invention and 95 parts of clay were mixed homogeneously, so that a dust formulation was prepared.

[Example 13] Rice blast control effect test (spray test)

A diluted solution of a wettable powder prepaired at 200 ppm in accordance with Example 9 was applied to

rice pots (variety: Koshihikari; 2-leaf stage) and air-dried. The plants were put into an artificial weather room (set conditions: 22°C, 12-hour dark light cycle) and spray-inoculated with a suspension of *Pyricularia oryzae* spores. The artificial weather room was kept at high humidity, and the examination was conducted after 7days.
The preventive value was calculated by the following equation, and was expressed based on the criteria shown in Table 3 below. The results are shown in Table 4-1 and Table 4-2.

Table 3

$$\text{Preventive value (\%)} = \{1-(\text{the number of lesions in treated plot})/(\text{the number of lesions in untreated plot})\} \times 100$$

| Effect | Preventive value |
|---|---|
| A | 80% or more |
| B | 50% or more and less than 80% |
| C | less than 50% |

The compounds represented by the following Formula (7) and Formula (8) included in WO2003008372 were used as reference chemical agents.

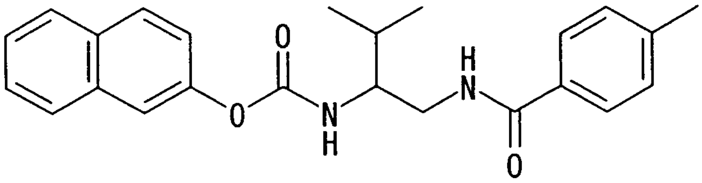
(7)

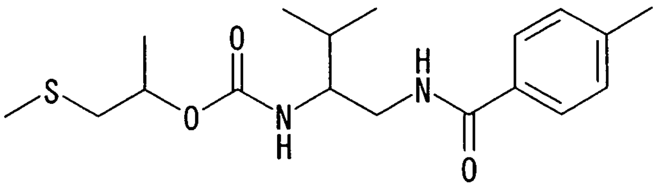
(8)

Table 4-1 Rice blast control effect test (spray test)

| Compound No. | Effect |
|---|---|
| 2 | A |
| 3 | A |
| 4 | A |
| 5 | A |
| 6 | A |
| 7 | A |
| 8 | A |

(continued)

| Compound No. | Effect |
|---|---|
| 9 | A |
| 10 | A |
| 11 | B |
| 13 | A |
| 14 | B |
| 15 | A |
| 16 | A |
| 17 | A |
| 24 | A |
| 25 | A |
| 28 | A |

Table 4-2 Rice blast control effect test (spray test)

| | |
|---|---|
| 30 | A |
| 31 | A |
| 32 | A |
| 33 | A |
| 34 | A |
| 35 | A |
| 36 | A |
| 37 | A |
| 38 | A |
| 39 | A |
| 40 | A |
| Formula (7) | C |
| Formula (8) | C |

[Example 14] Rice blast control effect test (paddy water application)

Rice plants (variety: Koshihikari; 3-leaf stage) were planted in 1/5000 are Wagner pots and grown for one week in a green house. Granules prepared in accordance with Example 7 were applied to water surface at a rate of 1.5 kg / 10 ares. After 30 days from the application, a liquid suspension of *Pyricularia* oryzae spores was spray-inoculated to the rice plants. The pots were placed under the conditions of 25°C and high humidity for 1 week, and the number of lesions was examined.
The preventive value was calculated by the following equation, and was expressed based on the criteria shown in Table 5 below. The results are shown in Table 6 (Table 6). The compounds similar to those in Example 13 were used as reference chemical agents.

Table 5

preventive value (%) = {1-(the number of lesions in treated plot)/(the number of lesions in untreated plot)} × 100

| Effect | Preventive value |
|---|---|
| A | 80% or more |
| B | 50% or more and less than 80% |
| C | less than 50% |

Table 6 Rice blast control effect test (application on water surface)

| Compound No. | Effect |
|---|---|
| 1 | A |
| 2 | A |
| 6 | A |
| 15 | A |
| 24 | A |
| 25 | A |
| 28 | A |
| 30 | A |
| 31 | A |
| Formula (7) | C |
| Formula (8) | C |

The diamine derivative according to the present invention exerts an excellent effect of controlling the rice blast and, therefore, is useful as a plant disease control agent.

**Claims**

1. A diamine derivative represented by Formula (1):

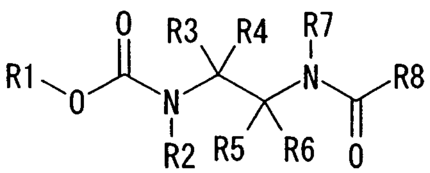 (1)

[In Formula, R1 represents a halogenated hydrocarbon having the carbon number of 1 to 6; R2 and R7 independently represent a hydrogen atom, a hydrocarbon having the carbon number of 1 to 6, or an acyl group; R3 and R4 independently represent a hydrogen atom, a hydrocarbon which has the carbon number of 1 to 6 and , which may be substituted, or a heteroaryl group which may be substituted, or represent a cycloalkyl group having the carbon number of 3 to 6 including a carbon atom bonding to R3 and R4; R5 and R6 independently represent a hydrogen atom or a hydrocarbon having the carbon number of 1 to 6; and R8 represents an arylalkyl group which may be

substituted, an aryl group which may be substituted, or a heteroaryl group which may be substituted.].

2. The diamine derivative according to Claim 1, wherein R1 represents a halogenated alkyl group having the carbon number of 1 to 6, a halogenated cycloalkyl group having the carbon number of 3 to 6, a halogenated alkenyl group having the carbon number of 2 to 6, or a halogenated cycloalkenyl group having the carbon number of 3 to 6; R2 and R7 independently represent a hydrogen atom, an alkyl group having the carbon number of 1 to 6, a cycloalkyl group having the carbon number of 3 to 6, an alkenyl group having the carbon number of 2 to 6, a cycloalkenyl group having the carbon number of 3 to 6, an alkynyl group having the carbon number of 2 to 6, an arylalkyl group which may be substituted, an aryl group which may be substituted, or an acyl group; R3 and R4 independently represent a hydrogen atom, an alkyl group which has the carbon number of 1 to 6 and which may be substituted, a cycloalkyl group which has the carbon number of 3 to 6 and which may be substituted, an alkenyl group having the carbon number of 2 to 6, a cycloalkenyl group having the carbon number of 3 to 6, an alkynyl group having the carbon number of 2 to 6, an arylalkyl group which may be substituted, a heteroarylalkyl group which may be substituted, an aryl group which may be substituted, or a heteroaryl group which may be substituted, or represent a cycloalkyl group having the carbon number of 3 to 6 including a carbon atom bonding to R3 and R4; R5 and R6 independently represent a hydrogen atom, an alkyl group having the carbon number of 1 to 6, a cycloalkyl group which has the carbon number of 3 to 6, an alkenyl group having the carbon number of 2 to 6, a cycloalkenyl group having the carbon number of 3 to 6, an alkynyl group having the carbon number of 2 to 6, an arylalkyl group which may be substituted, or an aryl group which may be substituted; and R8 represents an arylalkyl group which may be substituted, an aryl group which may be substituted, or a heteroaryl group which may be substituted.

3. The diamine derivative according to Claim 2, wherein R2 and R7 independently represent a hydrogen atom, an alkyl group having the carbon number of 1 to 6, a cycloalkyl group having the carbon number of 3 to 6, an arylalkyl group which may be substituted, an aryl group which may be substituted, or an acyl group; R3 and R4 independently represent a hydrogen atom, an alkyl group which has the carbon number of 1 to 6 and which may be substituted, a cycloalkyl group which has the carbon number of 3 to 6 and which may be substituted, an alkenyl group having the carbon number of 2 to 6, an arylalkyl group which may be substituted, a heteroarylalkyl group which may be substituted, an aryl group which may be substituted, or a heteroaryl group which may be substituted, or represent a cycloalkyl group having the carbon number of 3 to 6 including a carbon atom bonding to R3 and R4; and R5 and R6 independently represent a hydrogen atom, an alkyl group having the carbon number of 1 to 6, a cycloalkyl group which has the carbon number of 3 to 6, an arylalkyl group which may be substituted, or an aryl group which may be substituted.

4. The diamine derivative according to Claim 3, wherein R2 and R7 independently represent a hydrogen atom, an alkyl group having the carbon number of 1 to 6, or an acyl group; R3 and R4 independently represent a hydrogen atom, an alkyl group which has the carbon number of 1 to 6 and which may be substituted, a cycloalkyl group which has the carbon number of 3 to 6 and which may be substituted, an arylalkyl group which may be substituted, or an aryl group which may be substituted, or represent a cycloalkyl group having the carbon number of 3 to 6 including a carbon atom bonding to R3 and R4; and R5 and R6 independently represent a hydrogen atom or an alkyl group having the carbon number of 1 to 6.

5. The diamine derivative according to Claim 4, wherein each of R2, R5, R6, and R7 is a hydrogen atom.

6. A plant disease control agent comprising the diamine derivative according to any one of Claim 1 to Claim 5 as an active ingredient.

7. A process for producing the diamine derivative according to Claim 1, comprising reacting a compound represented by Formula (2):

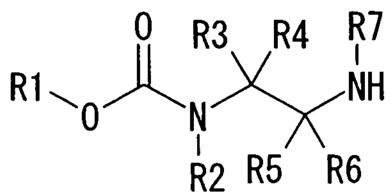

(2)

[In Formula, R1, R2, R3, R4, R5, R6, and R7 represent the same substances as those in Claim 1.] with a compound represented by Formula (3):

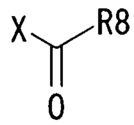
(3)

[In Formula, R8 represents the same substance as that in claim 1, and X represents a leaving group.].

**8.** A process for producing the diamine derivative according to Claim 1, comprising condensing a compound represented by Formula (2) and a compound represented by Formula (4):

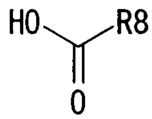
(4)

[In Formula, R8 represents the same substance as that in Claim 1.].

**9.** A process for producing the diamine derivative according to Claim 1, comprising reacting a compound represented by Formula (5):

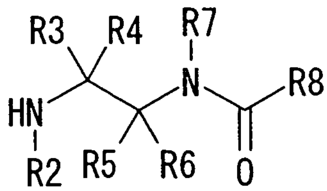
(5)

[In Formula, R2, R3, R4, R5, R6, R7, and R8 represent the same substances as those in Claim 1.] with a compound represented by Formula (6):

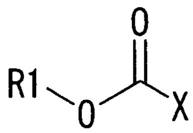
(6)

[In Formula, R1 represents the same substance as that in Claim 1, and X represents a leaving group.].

**INTERNATIONAL SEARCH REPORT**

International application No.
PCT/JP2004/015471

A. CLASSIFICATION OF SUBJECT MATTER
Int.Cl[7] C07C271/20, 271/34, 269/06, C07D277/68, 307/85, C07D333/60, A01N47/12

According to International Patent Classification (IPC) or to both national classification and IPC

B. FIELDS SEARCHED

Minimum documentation searched (classification system followed by classification symbols)
Int.Cl[7] C07C271/00, 269/00, C07D277/00, 307/00, 333/00, A01N47/00

Documentation searched other than minimum documentation to the extent that such documents are included in the fields searched

Electronic data base consulted during the international search (name of data base and, where practicable, search terms used)
REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| X | WO 01/46152 A1 (Monsanto Co.), 28 June, 2001 (28.06.01), & US 2002/52295 A1 | 1,7-8 |
| X | JP 2003-96046 A (Mitsui Chemicals, Inc.), 03 April, 2003 (03.04.03), Pages 4 to 9 & WO 03/8372 A1 & EP 1408027 A1 | 1-8 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 January, 2005 (06.01.05) | 25 January, 2005 (25.01.05) |
| Name and mailing address of the ISA/ Japanese Patent Office | Authorized officer |
| Facsimile No. | Telephone No. |

Form PCT/ISA/210 (second sheet) (January 2004)